# EUROPEAN PATENT APPLICATION

(11) **EP 4 725 551 A1**
(43) Date of publication of application: **15.04.2026**
(21) Application number: 24830553.4
(22) Date of filing: 18.06.2024
(51) Int. Cl.: A61P 27/02, C07K 19/00, C07K 1/06, A61K 38/16, A61K 38/39

(54) **FUSION POLYPEPTIDE AJ007 AND USE THEREOF**

(30) Priority: 26.06.2023 CN 202310763131
(71) Applicant: Nanjing Anji Biotechnology Co., Ltd., Nanjing, Jiangsu 210000 (CN)
(72) Inventor: XU, Hanmei, Nanjing, Jiangsu 210000 (CN); CHEN, Shujian, Nanjing, Jiangsu 210000 (CN)
(74) Representative: karo IP
(86) International application number: PCT/CN2024/099701
(87) International publication number: WO 2025/001907

(57) **Abstract**

This application discloses a fusion polypeptide AJ007 and use thereof, and belongs to the technical field of ophthalmic drugs. The fusion polypeptide AJ007 is formed by linking a TAT cell-penetrating peptide to a front end of a polypeptide HM-1 molecule that inhibits neovascularization. The fusion polypeptide AJ007 is used for preparing polypeptide eye drops. By selecting a prescription of this application, the fusion polypeptide AJ007 is prepared into eye drops. When a wet age-related macular degeneration disease is prevented or treated, an administration route of the eye drops can effectively avoid the problems of various serious side effects and poor patient tolerance caused by intravitreal injection clinically, has the advantage of non-invasive treatment, and exhibits excellent efficacy of improving abnormal proliferation of choroidal neovascularization in the laser-induced wAMD mouse model, thereby having good clinical treatment potential.

## Description

### TECHNICAL FIELD

This application belongs to the technical field of ophthalmic drugs, particularly relates to a fusion polypeptide AJ007 and use thereof, and more particularly relates to a fusion polypeptide AJ007 and use thereof in preparation of a drug for preventing or treating neovascular diseases in the posterior segment of ocular.

### BACKGROUND

With the increase of age, the aggravation of myopia, or the development of related metabolic diseases, when fundus oculi diseases, such as pathological myopia, age-related macular degeneration, and diabetic retinopathy occur, some unnecessary blood vessels are newly formed in the fundus oculi. These newly formed blood vessels invade originally normal fundus oculi tissues, such as choroidal blood vessels and retinal photoreceptor layers, resulting in impairment of visual functions and also blindness in severe cases.

The age-related macular degeneration (AMD) is a most common cause of irreversible visual impairment, including dry (non-exudative or atrophic) and wet (neovascular or exudative) types. Wet age-related macular degeneration (wAMD) is an important cause of low vision and blindness in the population, with its incidence rate increasing year by year. Choroidal neovascularization (CNV) is a main pathological basis and a primary cause of impairment of visual functions. Therefore, it is highly necessary to develop appropriate drugs to alleviate the visual decline of patients by improving CNV.

A vascular endothelial growth factor (VEGF) has been confirmed to play an important role in the formation of CNV. Therefore, drugs currently used clinically for the wet age-related macular degeneration belong to antibody drugs targeting VEGF, such as ranibizumab, aflibercept, and conbercept. These drugs have a certain effect of improving the vision of patients with wet age-related macular degeneration. However, such antibody drugs have relatively large molecular weights and cannot pass through a blood-ocular barrier to reach the retina and choroid. Therefore, intravitreal injection is clinically adopted as an administration route for treatment. However, the intravitreal injection leads to a variety of serious side effects, such as intraocular hemorrhage, intraocular infection, retinal detachment, intraocular inflammation, cataract, and elevated intraocular pressure, resulting in very poor patient compliance and tolerance. Therefore, more appropriate therapeutic regimens are needed clinically to avoid the occurrence of these serious side effects. In addition, such antibody drugs directly neutralize VEGF. However, it is reported in some documents that VEGF has a nutritional effect on the retina and choroid. If there is a long-term deficiency of VEGF, atrophy of the retina and choroid is likely to be caused, which may instead increase the risk of visual impairment. Therefore, it is more suitable to achieve treatment by targeting receptors of VEGF or other signaling pathways that inhibit neovascularization at the choroid.

In the applicant's prior Chinese invention patent with authorization announcement No. CN104045718B, a multifunctional fusion polypeptide VI (HM-1) is disclosed, with an amino acid sequence of Arg-Gly-Ala-Asp-Arg-Ala-Gly-Gly-Gly-Gly-Arg-Gly-Asp (SEQ ID NO: 1). The multifunctional fusion polypeptide VI has a higher ability to inhibit neovascularization, which mainly inhibits the formation of blood vessels by suppressing VEGFR2 and αvβ3 signaling pathways. Moreover, it is further verified that the multifunctional fusion polypeptide VI can treat choroidal neovascularization in rats. However, the multifunctional fusion polypeptide VI still requires administration via intravitreal injection. This administration route is not a better way for patient compliance clinically and may also lead to other serious side effects.

Therefore, it is necessary to optimize the multifunctional fusion polypeptide VI through appropriate method to reduce side effects, improve patient compliance, and provide a more appropriate clinical therapeutic regimen for patients with wet age-related macular degeneration.

### SUMMARY

### 1. Problems to be solved

In view of the problems that polypeptide drugs, such as a multifunctional fusion polypeptide VI (HM-1), in the prior art cannot pass through a blood-ocular barrier and need to be administered by intravitreal injection, and that the route easily causes serious side effects, this application provides a fusion polypeptide AJ007 and use thereof. The fusion polypeptide AJ007 is prepared by linking a cell-penetrating peptide TAT to a front end of a multifunctional fusion polypeptide VI (HM-1) sequence that has a higher ability to inhibit neovascularization, thereby enhancing its ability to penetrate through the blood-ocular barrier or sclera to reach the posterior segment of ocular. Moreover, the fusion polypeptide AJ007 and pharmaceutically acceptable salts, esters, or other forms thereof are used as a polypeptide bulk drug and combined with an appropriate auxiliary material selected to prepare eye drops for preventing and/or treating wet age-related macular degeneration and other ocular diseases, which not only enhances the efficacy of the fusion polypeptide AJ007, but also reduces side effects and facilitates clinical use.

### 2. Technical solutions

In order to solve the above problems, the following technical solutions are adopted in this application.

This application provides a fusion polypeptide AJ007, where the fusion polypeptide AJ007 has the following amino acid sequence: Tyr-Gly-Arg-Lys-Lys-Arg-Arg-Gln-Arg-Arg-Arg-Arg-Gly-Ala-Asp-Arg-Ala-Gly-Gly-Gly-Gly-Arg-Gly-Asp (YGRKKRRQRRRRGADRAGGGGRGD, SEQ ID NO: 2). By linking a cell-penetrating peptide TAT to a front end of a multifunctional fusion polypeptide VI (HM-1) sequence that has a higher ability to inhibit neovascularization disclosed previously by the applicant, its ability to penetrate through the blood-ocular barrier or sclera to reach the posterior segment of ocular is enhanced, so that the fusion polypeptide can penetrate through the blood-ocular barrier or sclera to reach the choroid.

This application further provides use of the above-mentioned fusion polypeptide AJ007 in preparation of a drug for preventing or treating neovascular diseases in the posterior segment of ocular, where the drug can inhibit neovascularization.

Further, the above-mentioned drug for preventing or treating the neovascular diseases in the posterior segment of ocular at least includes the fusion polypeptide AJ007 and/or a pharmaceutically acceptable carrier thereof.

Further, the above-mentioned drug for preventing or treating the neovascular diseases in the posterior segment of ocular includes eye drops containing the fusion polypeptide AJ007 and/or a pharmaceutically acceptable carrier thereof.

Further, the above-mentioned neovascular diseases in the posterior segment of ocular include: one or more of wet age-related macular degeneration (wAMD), retinal vein occlusion (RVO), diabetic retinopathy (DR), retinopathy of prematurity (ROP), and other diseases, where the diabetic retinopathy, the retinal vein occlusion, and the retinopathy of prematurity are diseases highly associated with abnormal neovascularization.

The diabetic retinopathy (DR) is a chronic progressive disease that worsens with the progression of diabetes mellitus, which may be manifested as retinal exudation, hemorrhage, macular edema, and retinal neovascularization. When a lesion develops to a certain extent, varying degrees of hypopsia occur, and in severe cases, blindness can be caused. The main pathogenesis of DR is that a continuous hyperglycemic environment damages a blood-retina barrier to increase the permeability and cause microvascular permeability, leading to abnormal neovascularization and fibroproliferation of the retina, and ultimately resulting in structural changes or even complete loss of retinal microvascular cells. The level of VEGF is closely related to the severity of DR, and vision loss is reduced by controlling or delaying the lesion progression through anti-VEGF drug therapy.

The retinal vein occlusion (RVO) is a common retinal vascular disease caused by the obstruction of blood flow in retinal veins, which is mainly manifested as the inability of the veins to drain retinal blood and exudation of retinal vessels, leading to macular edema and intraretinal hemorrhage, and resulting in decreased vision. The pathogenesis of RVO is diverse, mainly including changes in the vascular wall, hemorheological changes, and hemodynamic changes. There are no specific treatment methods for RVO, and current treatment method cannot effectively reverse the pathological changes of retinal venous obstruction. However, the administration of anti-VEGF drugs for treatment can inhibit the macular edema and neovascularization caused by RVO, thereby maintaining the vision of patients.

The retinopathy of prematurity (ROP) is a fundus oculi disease of premature infants with pathological neovascularization proliferation accompanied by fibrotic changes, which is a main cause of blindness in children worldwide. A main pathological mechanism is abnormal proliferation of retinal vessels caused by retinal vascular occlusion and hypoxia after birth. Anti-VEGF therapy is a better approach for treating ROP.

The pathological manifestations and pathogenesis of DR, RVO, and ROP have many similarities to wAMD. Moreover, it is proven that anti-VEGF treatment has a good ability to control the conditions of DR, RVO, and ROP. Therefore, AJ007 also has great potential for improving the courses of DR, RVO, and ROP by inhibiting abnormal vascularization of the retina.

Further, the above-mentioned neovascular disease in the posterior segment of ocular includes the wet age-related macular degeneration.

This application further provides a polypeptide eye drops for preventing or treating neovascular diseases in the posterior segment of ocular, where the polypeptide eye drops include the above-mentioned fusion polypeptide AJ007 or a pharmaceutically acceptable salt form thereof, and are combined with an auxiliary material.

Further, the above-mentioned polypeptide eye drops for preventing or treating neovascular diseases in the posterior segment of ocular include the following components per 100 mL:
0-50% w/v of the fusion polypeptide AJ007 or a pharmaceutically acceptable salt form thereof;
5-10% w/v of 2-hydroxypropyl-β-cyclodextrin;
0.1-5% w/v of polysorbate 80;
0.05-5% w/v of tyloxapol;
0.05-10% w/v of poloxamer 407;
0-1% w/v of sodium dihydrogen phosphate;
0-0.005% w/v of a preservative;
0-1% w/v of an osmotic pressure regulator for regulating an osmotic pressure molar concentration to 250-350 mOsm/kg; and
0-1% w/v of a pH regulator for regulating a pH to 6.0-8.0.

In the polypeptide eye drops, by using the fusion polypeptide AJ007 or a pharmaceutically acceptable salt form thereof as a polypeptide bulk drug and combining with the auxiliary material as a carrier of the fusion polypeptide AJ007 or a pharmaceutically acceptable salt form thereof, the inclusion ability of the polypeptide bulk drug can be effectively increased, thereby promoting the dissolution of the fusion polypeptide AJ007, enhancing the stability of the fusion polypeptide AJ007, and making the fusion polypeptide to have a better effect of penetrating through the blood-ocular barrier or sclera to reach the posterior segment of ocular. Through the optimization of the two aspects, including linking the cell-penetrating peptide TAT to the front end of the multifunctional fusion polypeptide VI (HM-1) sequence that has a higher ability to inhibit neovascularization and combining with the auxiliary material selected in this application, the fusion polypeptide AJ007 with an anti-neovascularization function is delivered to the retina to the choroid in the posterior segment of ocular, which can improve CNV by an administration route of eye dropping.

Further, the above-mentioned polypeptide eye drops for preventing or treating neovascular diseases in the posterior segment of ocular include the following components per 100 mL:
2% w/v of the fusion polypeptide AJ007 or a pharmaceutically acceptable salt form thereof;
8.41% w/v of the 2-hydroxypropyl-β-cyclodextrin;
1% w/v of the polysorbate 80;
0.1% w/v of the tyloxapol;
2% w/v of the poloxamer 407;
0.142% w/v of the sodium dihydrogen phosphate;
0.005% w/v of the preservative;
the osmotic pressure regulator for regulating the osmotic pressure molar concentration to 276 mOsm/kg; and
the pH regulator for regulating the pH to 6.47.

Further, the pharmaceutically acceptable salt form of the above-mentioned fusion polypeptide AJ007 includes one or more of an acetate form, a trifluoroacetate form, a hydrochloride form, an ammonium salt form, a sodium salt form, a dihydroxynaphthalate form, a citrate form, and a salicylate form. Furthermore, the pharmaceutically acceptable salt form of the above-mentioned fusion polypeptide AJ007 includes the acetate form.

Further, the above-mentioned preservative includes one or more of benzalkonium chloride, a benzalkonium bromide cationic surfactant, benzyl alcohol, chlorobutanol, and sorbic acid. Furthermore, the above-mentioned preservative includes the benzalkonium chloride at a proportion of 0.005% w/v.

Further, the above-mentioned osmotic pressure regulator includes one or more of sodium chloride, boric acid, and glucose. Furthermore, the above-mentioned osmotic pressure regulator includes the sodium chloride at a proportion of 0.2% w/v.

Further, the above-mentioned pH regulator includes one or more of a phosphate buffer solution, a boric acid buffer solution, hydrochloric acid, and sodium hydroxide. Furthermore, the above-mentioned pH regulator includes the sodium hydroxide at a proportion of 0.8% w/v.

Further, the above-mentioned neovascular disease in the posterior segment of the eye includes one or more of wet age-related macular degeneration, retinal vein occlusion, diabetic retinopathy, retinopathy of prematurity, and other diseases.

Further, the above-mentioned neovascular disease in the posterior segment of the eye includes the wet age-related macular degeneration.

This application further provides a method for preparing the above-mentioned polypeptide eye drops for preventing or treating neovascular diseases in the posterior segment of ocular, which specifically includes the following steps:
(1) preparation of an auxiliary material solution: weighing ultrapure water, sequentially adding specified amounts of the sodium dihydrogen phosphate, the benzalkonium chloride, the polysorbate 80, the poloxamer 407, and the tyloxapol, stirring continuously until complete dissolution, and filtering with a 0.22 µm filter membrane to obtain a solution serving as solution A for later use;
(2) preparation of a 2-hydroxypropyl-β-cyclodextrin solution: weighing the solution A, placing the same in a water bath stirring pot, heating up to 60°C, starting stirring, adding the 2-hydroxypropyl-β-cyclodextrin in several portions, and after complete dispersion and dissolution, taking out a solution and cooling to serve as solution B for later use;
(3) regulation of pH and osmotic pressure: detecting a pH and an osmotic pressure of the solution B, regulating the pH and the osmotic pressure using the pH regulator and the osmotic pressure regulator, and filtering with a 0.22 µm filter membrane to obtain a solution serving as solution C for later use; and
(4) preparation of the polypeptide eye drops: weighing and adding the fusion polypeptide AJ007 or a pharmaceutically acceptable salt form thereof to the solution C, and shaking continuously to completely disperse and dissolve the fusion polypeptide AJ007 or a pharmaceutically acceptable salt form thereof to obtain the polypeptide eye drops.

### 3. Beneficial effects

Compared with the prior art, this application has the following beneficial effects.
(1) According to the fusion polypeptide AJ007 provided by this application, by linking the cell-penetrating peptide TAT to the front end of the multifunctional fusion polypeptide VI (HM-1) sequence that has a higher ability to inhibit neovascularization disclosed previously by the applicant, its ability to penetrate through the blood-ocular barrier or sclera to reach the posterior segment of ocular is enhanced, so that the fusion polypeptide can penetrate through the blood-ocular barrier or sclera to reach the choroid. Moreover, it can be seen from Example 3 that only less than 10 ng/mL of active molecules of the original multifunctional fusion polypeptide VI (HM-1) can reach the lesion target site of wAMD, namely RPE/choroid complex, while 6291.38 ng/mL of active molecules of the fusion polypeptide AJ007 can reach the RPE/choroid complex, thereby greatly improving the ocular penetration ability of the original active molecules. According to existing technical solutions, after a polypeptide AT7 targeting VEGFR2 is linked to the cell-penetrating peptide TAT, its ability to penetrate through and enter endothelial cells is increased by 119 times; after bevacizumab is linked to a cell-penetrating peptide CPP, its ability to penetrate through and enter the posterior segment of the eye after eye dropping is increased by approximately 10 times; in patent CN107129521A, after a cell-penetrating peptide CC12 is linked to KV12, its ability to penetrate through and enter the eye after eye dropping is increased by approximately 8 times; in patent CN114073774A, after a cell-penetrating peptide 289WP is linked to ranibizumab, its concentration reaching the retina after eye dropping is increased by approximately 100 times; and in patent CN114668717A, after a cell-penetrating peptide PENE is linked to axitinib, its concentration reaching the retina after eye dropping is increased by approximately 5-10 times. However, compared with HM-1, the ability to penetrate through the posterior segment of ocular of AJ007 is increased by approximately 600 times in comparison to the above-mentioned existing technical solutions. The superiority of AJ007 is still obvious, and this superiority exceeds a normal expectation, indicating the particularity and creativity of the peptide.
(2) The polypeptide eye drops for preventing or treating neovascular diseases in the posterior segment of ocular provided by this application are prepared using the fusion polypeptide AJ007 or a pharmaceutically acceptable salt form thereof as a polypeptide bulk drug and combining with an appropriate auxiliary material selected. Through the optimization of the two aspects, including linking the cell-penetrating peptide TAT to the front end of the multifunctional fusion polypeptide VI (HM-1) sequence that has a higher ability to inhibit neovascularization and combining with the auxiliary material selected in this application, a brand-new composition product is ultimately obtained, so that the product is distinguished from other peptide eye drops and has unique novelty. The fusion polypeptide AJ007 with an anti-neovascularization function can be delivered to the retina/choroid in the posterior segment of ocular, which can improve CNV by an administration route of eye dropping. By comparing with the original multifunctional fusion polypeptide VI (HM-1) in ocular tissue drug distribution and laser-induced mouse CNV efficacy experiments, experimental results of the ocular tissue drug distribution show that compared with the multifunctional fusion polypeptide VI (HM-1), the polypeptide eye drops of this application have at least 100 times or more of active molecules that can reach the lesion target site of wAMD, namely the RPE/choroid complex. Moreover, efficacy experiment results show that the polypeptide eye drops of this application have an extremely superior effect of improving CNV leakage compared with the original multifunctional fusion polypeptide VI (HM-1) molecule, and also have non-inferiority even compared with aflibercept as a currently most effective wAMD treatment drug clinically, which is difficult for all other peptide eye drops to achieve, is more advanced in technology, and overcomes the shortcoming of limited efficacy of previous eye drops for treating fundus oculi diseases, thereby having stronger clinical potential for the treatment of wAMD. The polypeptide eye drops are expected to change the current situation that wAMD patients need to accept the treatment method of intravitreal injection, which can significantly increase the compliance of patients and provide a treatment option capable of significantly improving the compliance of the wAMD patients.

### BRIEF DESCRIPTION OF THE DRAWINGS

FIG. 1 shows drug-time curve results of an original multifunctional fusion polypeptide VI (HM-1) after eye dropping.
FIG. 2 shows drug-time curve results of polypeptide eye drops of this application after eye dropping.
FIG. 3 shows schematic diagrams of fundus fluorescein angiography (FFA) after administration of the polypeptide eye drops of this application to the wAMD mouse model.
FIG. 4 shows a statistical chart of CNV leakage areas in FFA after the administration of the polypeptide eye drops of this application to the wAMD mouse model.
FIG. 5 shows schematic diagrams of fluorescein isothiocyanate (FITC)-dextran flat-mount staining after the administration of the polypeptide eye drops of this application to the wAMD mouse model.
FIG. 6 shows a statistical chart of CNV areas in the (FITC)-dextran flat-mount staining after the administration of the polypeptide eye drops of this application to the wAMD mouse model.
FIG. 7 shows drug-time curve results of the original multifunctional fusion polypeptide VI (HM-1) at an equimolar concentration after eye dropping.
FIG. 8 shows drug-time curve results of an original multifunctional fusion polypeptide VI (HM-1) preparation at an equimolar concentration after eye dropping.
FIG. 9 shows drug-time curve results of a fusion polypeptide AJ007 at an equimolar concentration after eye dropping.
FIG. 10 shows drug-time curve results of the polypeptide eye drops of this application at an equimolar concentration after eye dropping.
FIG. 11 shows drug-time curve results of the original multifunctional fusion polypeptide VI (HM-1) at an equimolar concentration after intravitreal injection.
FIG. 12 shows schematic diagrams of FFA after administration of various drug solutions at an equimolar concentration to the wAMD mouse model.
FIG. 13 shows a statistical chart of CNV leakage areas in FFA after the administration of various drug solutions at an equimolar concentration to the wAMD mouse model.
FIG. 14 shows schematic diagrams of FITC-dextran flat-mount staining after the administration of various drug solutions at an equal molar concentration to the wAMD mouse model.
FIG. 15 shows a statistical graph of CNV areas in the FITC-dextran flat-mount staining after the administration of various drug solutions at an equimolar concentration to the wAMD mouse model.

### DETAILED DESCRIPTION

This application is further described below in conjunction with specific examples.

It should be noted that the terms such as "up", "down", "left", "right", and "middle" cited in this specification are only used for the sake of clarity in description and are not intended to limit the scope of implementation, and changes or adjustments in their relative relationships, without substantial alterations to the technical contents, should also be regarded as within the scope of implementation of this application.

Unless otherwise defined, all technical terms and scientific terms used herein have the same meanings as generally understood by those skilled in the technical field to which this application belongs; and the term "and/or" as used herein includes any and all combinations of one or more related listed items.

Those without specific conditions in the examples are used in accordance with conventional conditions or conditions recommended by manufacturers. All reagents or instruments used without specific manufacturers are conventional products that can be purchased on the market.

As used herein, the term "approximately" is used for providing flexibility and imprecision associated with a given term, measure, or value. The degree of flexibility of specific variables can be easily determined by those skilled in the art.

As used herein, the term "at least one of ..." aims to be synonymous with "one or more of ...". For example, "at least one of A, B, and C" explicitly includes only A, only B, only C, and their respective combinations.

Concentrations, quantities, and other numerical value data can be presented in the form of a range herein. It should be understood that the form of a range is used only for convenience and brevity, and should be interpreted flexibly to not only include numerical values that are explicitly stated as limits of the range, but also include all individual numerical values or subranges that are covered within the range, as if each numerical value and subrange are explicitly stated. For example, the numerical range from approximately 1 to approximately 4.5 should be interpreted to not only include explicitly stated limit values from 1 to approximately 4.5, but also include individual numbers (such as 2, 3, and 4) and subranges (such as 1 to 3 and 2 to 4). The same principle is applicable to ranges that state only one value, such as "less than approximately 4.5", which should be interpreted to include all of the above values and ranges. Moreover, the interpretation should be applied regardless of the scope or breadth of features described.

### Example 1

This example provides a fusion polypeptide AJ007.

The fusion polypeptide AJ007 has an amino acid sequence of Tyr-Gly-Arg-Lys-Lys-Arg-Arg-Gln-Arg-Arg-Arg-Arg-Gly-Ala-Asp-Arg-Ala-Gly-Gly-Gly-Gly-Arg-Gly-Asp, (YGRKKRRQRRRRGADRAGGGGRGD, SEQ ID NO: 2). Arg-Gly-Ala-Asp-Arg-Ala-Gly-Gly-Gly-Gly-Arg-Gly-Asp (SEQ ID NO: 1) is a multifunctional fusion polypeptide VI (HM-1) disclosed previously by the applicant in a Chinese invention patent with authorization announcement No. CN104045718B. The multifunctional fusion polypeptide VI has a higher ability to inhibit neovascularization, which mainly inhibits the formation of neovascularization by suppressing VEGFR2 and αvβ3 signaling pathways, and therefore can improve choroidal neovascularization and leakage in mice caused by laser induction.

### Example 2

This example provides use of the fusion polypeptide AJ007 of Example 1 in preparation of a drug for preventing or treating a neovascular disease in a posterior segment of an eye, where the drug is polypeptide eye drops for preventing or treating neovascular diseases in the posterior segment of ocular. This example further provides polypeptide eye drops for preventing or treating neovascular diseases in the posterior segment of ocular and a preparation method thereof.

The polypeptide eye drops for preventing or treating neovascular diseases in the posterior segment of ocular provided in this example include the following components per 100 mL:
2% w/v of an acetate form of the fusion polypeptide AJ007;
8.41% w/v of 2-hydroxypropyl-β-cyclodextrin;
1% w/v of polysorbate 80;
0.1% w/v of tyloxapol;
2% w/v of poloxamer 407;
0.142% w/v of sodium dihydrogen phosphate;
0.005% w/v of a preservative (benzalkonium chloride);
0.2% w/v of an osmotic pressure regulator (sodium chloride) for regulating an osmotic pressure molar concentration to 276 mOsm/kg; and
0.8% w/v of a pH regulator (sodium hydroxide) for regulating a pH to 6.47.

The preparation method of the polypeptide eye drops for preventing or treating neovascular diseases in the posterior segment of ocular provided in this example specifically includes the following steps:
(1) preparation of an auxiliary material solution: weighing 100 mL of ultrapure water, sequentially adding specified amounts of the sodium dihydrogen phosphate, the benzalkonium chloride, the polysorbate 80, the poloxamer 407, and the tyloxapol, stirring continuously until complete dissolution, and filtering with a 0.22 µm filter membrane to obtain a solution serving as solution A for later use;
(2) preparation of a 2-hydroxypropyl-β-cyclodextrin solution: weighing 100 mL of the solution A, placing the same in a water bath stirring pot, heating up to 60°C, starting stirring, adding the 2-hydroxypropyl-β-cyclodextrin in several portions, and after complete dispersion and dissolution, taking out a solution and cooling to serve as solution B for later use;
(3) regulation of pH and osmotic pressure: detecting a pH and an osmotic pressure of the solution B, respectively regulating the pH and the osmotic pressure to 7.5 and 250 using the pH regulator and the osmotic pressure regulator, and filtering with a 0.22 µm filter membrane to obtain a solution serving as solution C for later use;
(4) preparation of the polypeptide eye drops: weighing and adding 2,000 mg of the acetate form of the fusion polypeptide AJ007 to 100 mL of the solution C, and shaking continuously to completely disperse and dissolve the fusion polypeptide AJ007 or a pharmaceutically acceptable salt form thereof to obtain the AJ007 polypeptide eye drops with a concentration of 20 mg/mL, where the measured osmotic pressure molar concentration is 276 mOsm/kg, and the pH is 6.47.

### Example 3

This example provides an ocular tissue distribution comparison experiment of the polypeptide eye drops (AJ007 eye drops) in Example 2 with the multifunctional fusion polypeptide VI (HM-1), which is specifically described below.

Drug solutions: The polypeptide eye drops (AJ007 eye drops) contained 20 mg/mL of the fusion polypeptide AJ007; and a multifunctional fusion polypeptide VI (HM-1) solution contained 20 mg/mL of the multifunctional fusion polypeptide VI (HM-1).

Animal grouping: 24 female C57BL/6J mice were randomly divided into a total of 6 time point groups, including 0 h, 1 h, 3 h, 7 h, 12 h, and 24 h, with 2 mice in each group.

Experimental process: All the mice in the eye drop groups were administered bilateral eye drops 1 time, with 5 µL of the corresponding drug solution in each eye. At the 6 time points of 0 h, 1 h, 3 h, 7 h, 12 h, and 24 h after the completion of administration, the mice in the corresponding groups were respectively subjected to eyeball removal and blood collection, the collected whole blood was immediately centrifuged at 6,000 rpm for 5 minutes, and then, a supernatant was sucked and stored as a sample to be tested. Additionally, 3 tissue sites, namely the vitreous body, the retina, and the RPE/choroid complex, were isolated from an eyeball under a stereomicroscope, into which a phosphate buffer solution (PBS) was added at 1:10 for dilution, followed by grinding and centrifugation at 12,000 rpm for 5 minutes. A supernatant was sucked to serve as a sample to be tested. Subsequently, the concentration of the fusion polypeptide AJ007 or the multifunctional fusion polypeptide VI (HM-1) in each tissue was detected by an enzyme-linked immunosorbent assay (ELISA) method. The ELISA method is as follows:
(1) coating: diluting HM-1 with a certain concentration of a coating solution (CBS) to 1 µg/mL, adding the diluted solution into a microplate at 100 µL per well, and standing overnight at 4°C;
(2) plate washing: discarding the coating solution, adding 265 µL of a PBST washing solution to each well, washing 3 times for 3 minutes each time, and beating to dryness;
(3) blocking: adding 200 µL of a 1% BSA blocking solution to each well, placing the solution in a water bath at 37°C for 1.5 hours, repeating the plate washing process, and beating to dryness;
(4) addition of a sample to be tested and an antibody: first, adding 100 µL of the sample to be tested to a microplate, then adding 100 µL of an HM-1 monoclonal antibody dilution solution at a certain concentration, setting 2-3 replicate wells, shaking uniformly on a shaker at 37°C for 2 minutes, then transferring the mixture to a water bath pot for placement at 37°C for 2 hours, taking out the mixture after a thorough reaction, and repeating the plate washing process;
(5) reaction with an enzyme-labeled secondary antibody: adding a certain concentration of HRP enzyme-labeled goat anti-mouse IgG at 100 µL per well for incubation at 37°C for 1.5 hours, repeating the plate washing process, and beating to dryness;
(6) addition of a substrate: adding 100 µL of a TMB substrate chromogenic solution to each well for color development at room temperature in the dark for a certain period of time;
(7) termination: adding 50 µL of a termination solution to each well to terminate the reaction; and
(8) detection: reading an OD value of each well at 450 nm using a microplate reader.

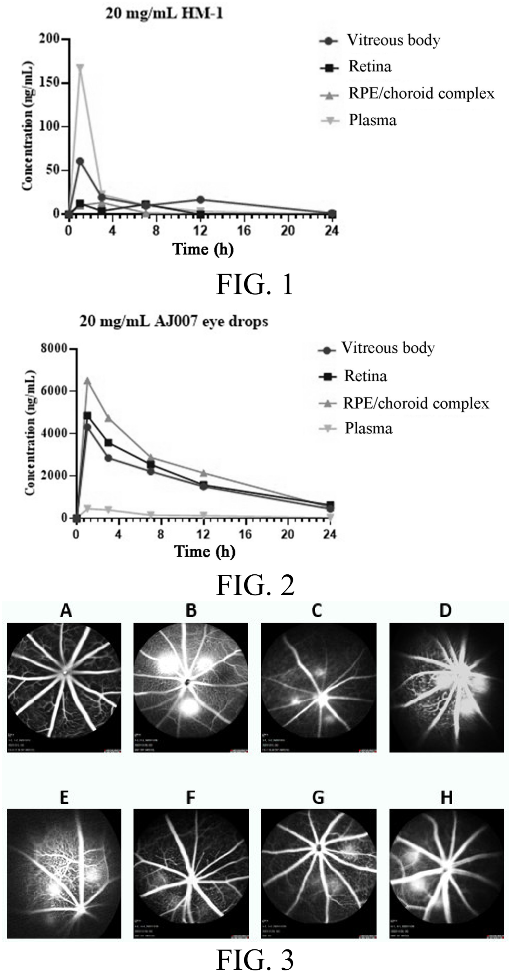

Experimental results and conclusions:
By comparing drug-time curve results of HM-1 after eye dropping and the AJ007 eye drops after eye dropping (FIG. 1 and FIG. 2), it can be found that only an extremely small number of active molecules of HM-1 can reach the lesion target site of wAMD, namely the RPE/choroid complex, after eye dropping, and the maintenance time is also relatively short. However, at least 100 times or more of active molecules of the AJ007 eye drops at the same concentration can reach the RPE/choroid complex after eye dropping, and there are still many active molecules present at this site at 24 hours, thereby greatly improving the bioavailability. The multifunctional fusion polypeptide VI (HM-1) cannot pass through a blood-ocular barrier to reach the RPE/choroid complex after eye dropping, and therefore cannot achieve the therapeutic effect of improving CNV leakage. However, by adding the TAT peptide, more active molecules can be promoted to reach the RPE/choroid complex. Compared with general cell-penetrating peptides linked to small molecules or large molecules that have the improvement effect of reaching the posterior segment of ocular only within several times (CN107129521A, CN114073774A, CN114668717A), a significant improvement is achieved.

### Example 4

This example provides an efficacy comparison experiment of the polypeptide eye drops (AJ007 eye drops) in Example 2 with the multifunctional fusion polypeptide VI (HM-1), which is specifically described below.

Animal grouping: 56 female C57BL/6J mice were randomly divided into a complete blank group, a model + vehicle (control solution containing all equal amounts of auxiliary material combinations but not containing the fusion polypeptide AJ007) control group, a model + positive drug aflibercept intravitreal injection (10 mg/mL) group, a model + low-dose HM-1 (20 mg/mL) group, a model + high-dose HM-1 (80 mg/mL) group, a model + low-dose AJ007 eye drop (20 mg/mL) group, a model + medium-dose AJ007 eye drop (40 mg/mL) group, and a model + high-dose AJ007 eye drop (80 mg/mL) group, with 7 mice in each group.

Experimental process: The mice were first treated with compound tropicamide eye drops for mydriasis, and then administered a 4% chloral hydrate solution by intraperitoneal injection for anesthesia at 0.2 mL/20 g of mouse body weight. After the anesthesia, a sodium hyaluronate gel was applied for eye protection, and a cover glass was attached. The left eyes were subjected to laser modeling using a 532 nm argon ion laser with a power of 120 mW, a blasting time of 0.01 seconds, and a spot size of 100 µm. Administration began on the day of modeling, which was only performed to the left eyes in various groups according to different administration routes respectively, where the mice in the eye drop groups were administered the eye drops 5 times a day, with an interval of 1 hour each time, and 5 µL of a corresponding drug solution was dropped into the eyes each time for 7 days. The mice in the intravitreal injection group were anesthetized on the day of modeling and then administered by intravitreal injection, with 1 µL of a corresponding drug solution injected into each mouse using a micro-syringe. After the modeling, a fundus fluorescein angiography instrument system was used to conduct an FFA examination on day 3 after the modeling to evaluate a CNV leakage level. On day 7 after the modeling, the mice were sacrificed, administered FITC-dextran by intraventricular injection, and then subjected to RPE-choroid/sclera complex flat-mount fluorescence staining to detect changes in CNV area.

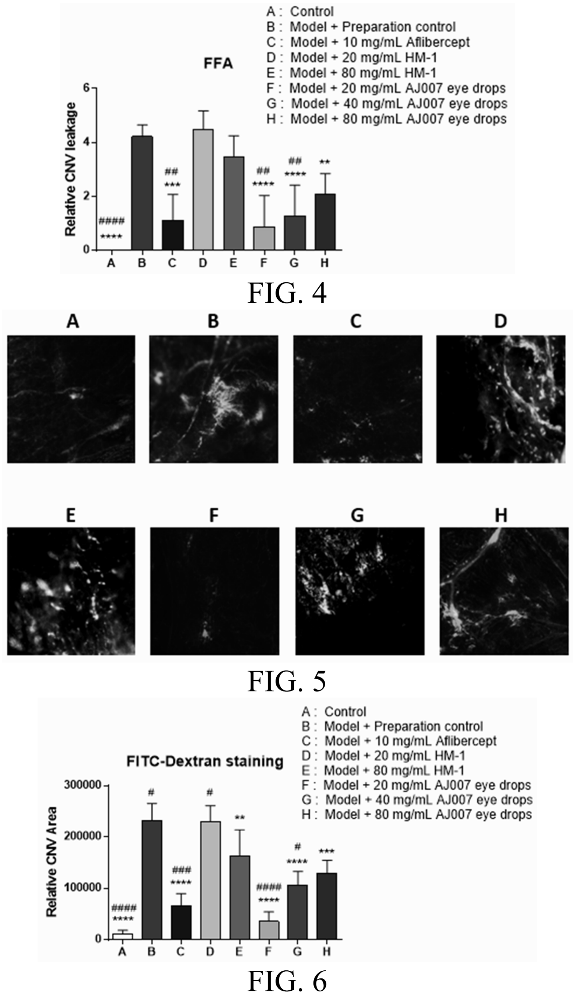

Experimental results and conclusions: FFA examination results show that the administration of low-dose or high-dose HM-1 cannot significantly improve laser-induced CNV leakage, while the administration of the low-dose AJ007 eye drops can significantly improve CNV leakage and is significantly better than low-dose and high-dose HM-1, and the effect is better than the medium-dose and high-dose AJ007 eye drops, which achieves efficacy on par with the intravitreal injection of positive drug aflibercept (FIG. 3 and FIG. 4). After the FITC-dextran injection, flat-mount staining results show that low-dose HM-1 cannot reduce the laser-induced CNV area, while high-dose HM-1 can partially reduce the CNV area. However, the administration of the low-dose AJ007 eye drops can greatly reduce the CNV area and is significantly better than high-dose HM-1, the effect is also better than that of the medium-dose and high-dose AJ007 eye drops, and the efficacy is even slightly superior to that of the intravitreal injection of positive drug aflibercept (FIG. 5 and FIG. 6). According to comprehensive results of the FFA examination and the flat-mount staining after the FITC-dextran injection, it can be seen that the AJ007 eye drops at 20 mg/mL have a very significant effect of improving abnormal neovascularization of the wAMD mouse model and have significantly better efficacy than low-dose and high-dose HM-1, and the efficacy in this classic wAMD model is on par with or even slightly better than that of the intravitreal injection of aflibercept, which is currently a treatment method with an optimal effect for wAMD clinically.

### Example 5

This example provides an ocular tissue distribution comparison experiment of the polypeptide eye drops (AJ007 eye drops) in Example 2 with an equimolar concentration of the multifunctional fusion polypeptide VI (HM-1), a multifunctional fusion polypeptide VI preparation (HM-1 preparation, with a polypeptide being HM-1 and a formulation of other auxiliary materials being the same as the AJ007 eye drops), a fusion polypeptide AJ007 preparation (TAT-HM-1, with a polypeptide being AJ007 and pure water as a vehicle) after eye dropping, and the multifunctional fusion polypeptide VI (HM-1) after intravitreal injection, which is specifically described below.

Animal grouping: 70 female C57BL/6J mice were randomly divided into a total of 7 time point groups, including 0 h, 10 min, 1 h, 3 h, 7 h, 12 h, and 24 h, with 2 mice in each group.

Experimental process: All the mice in the eye drop administration groups were administered bilateral eye drops 1 time, with 5 µL of a corresponding drug solution dropped into each eye, while 5 µL of a corresponding drug solution was injected into both eyes in the HM-1 intravitreal injection group. At the 7 time points of 0 h, 10 min, 1 h, 3 h, 7 h, 12 h, and 24 h after the completion of administration, the mice in the corresponding groups were respectively subjected to eyeball removal and blood collection, the collected whole blood was immediately centrifuged at 6,000 rpm for 5 minutes, and then, a supernatant was sucked and stored as a sample to be tested. Additionally, 3 tissue sites, namely the vitreous body, the retina, and the RPE/choroid complex, were isolated from an eyeball under a stereomicroscope, into which PBS was added at 1:10 for dilution, followed by grinding and centrifugation at 12,000 rpm for 5 minutes. A supernatant was sucked to serve as a sample to be tested. Subsequently, the concentration of HM-1 or fusion polypeptide AJ007 in each tissue was detected by an ELISA method.

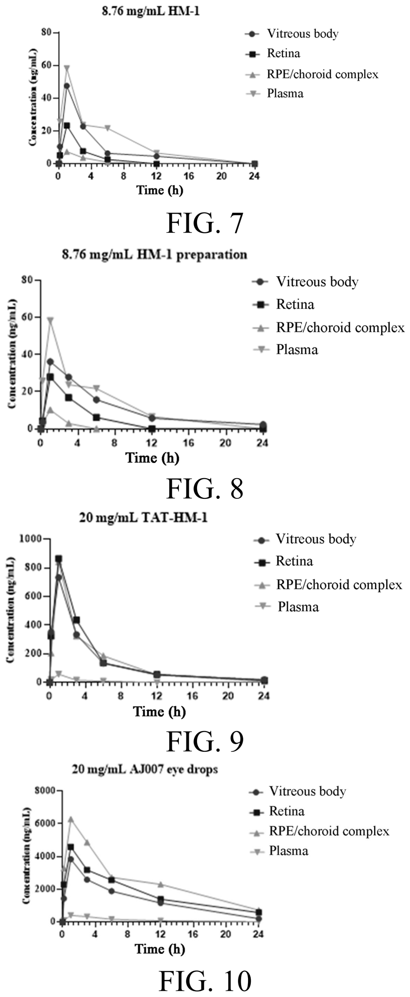

Experimental results and conclusions:
By comparing drug-time curve results of the AJ007 eye drops with the equimolar concentration of HM-1, HM-1 preparation, TAT-HM-1 after eye dropping, and HM-1 after intravitreal injection (FIG. 7, FIG. 8, FIG. 9, FIG. 10, and FIG. 11), it can be found that whether after eye dropping of HM-1 or HM-1 prepared into a preparation, only less than 10 ng/mL of active molecules can reach the lesion target site of wAMD, namely the RPE/choroid complex, and the maintenance time is also very short. However, after linking TAT to HM-1, 843.28 ng/mL of active molecules can reach the RPE/choroid complex, with a concentration much higher than that of the HM-1 and HM-1 preparation groups, but the maintenance time is also shorter. After the intravitreal injection of HM-1, approximately 5729.7 ng/mL of active molecules can reach the RPE/choroid complex, with a concentration higher than that of the TAT-HM-1 group, but the maintenance time is shorter. In contrast, after the eye dropping of the AJ007 eye drops, approximately 6291.38 ng/mL of active molecules can reach the RPE/choroid complex, which is much higher than that of the HM-1, HM-1 preparation, and TAT-HM-1 eye drop groups, and is slightly higher than that of the HM-1 intravitreal injection group, and the maintenance time is also longer than that of the HM-1 intravitreal injection group, indicating that the AJ007 eye drops prepared after optimization and modification can enable a large number of active molecules to reach the RPE/choroid complex, thereby providing a pharmacokinetic data support for the efficacy of effectively improving abnormal neovascularization at the choroid.

### Example 6

This example provides an efficacy comparison experiment of the polypeptide eye drops (AJ007 eye drops) in Example 2 with an equimolar concentration of the multifunctional fusion polypeptide VI (HM-1), a multifunctional fusion polypeptide VI preparation (HM-1 preparation), a fusion polypeptide AJ007 (TAT-HM-1) after eye dropping, and the multifunctional fusion polypeptide VI (HM-1) after intravitreal injection, which is specifically described below.

Animal grouping: 42 female C57BL/6J mice were randomly divided into a model + vehicle control group, a model + positive drug aflibercept intravitreal injection (10 mg/mL) group, a model + HM-1 (8.76 mg/mL) group, a model + HM-1 preparation (8.76 mg/mL) group, a model + TAT-HM-1 (20 mg/mL) group, a model + AJ007 eye drop (20 mg/mL) group, and a model + HM-1 intravitreal injection (8.76 mg/mL) group, with 6 mice in each group.

Experimental process: The mice were first treated with compound tropicamide eye drops for mydriasis, and then administered a 4% chloral hydrate solution by intraperitoneal injection for anesthesia at 0.2 mL/20 g of mouse body weight. After the anesthesia, a sodium hyaluronate gel was applied for eye protection, and a cover glass was attached. The left eyes were subjected to laser modeling using a 532 nm argon ion laser with a power of 120 mW, a blasting time of 0.01 seconds, and a spot size of 100 µm. Administration began on the day of modeling, which was only performed to the left eyes in various groups according to different administration routes respectively, where the mice in the eye drop groups were administered the eye drops 5 times a day, with an interval of 1 hour each time, and 5 µL of a corresponding drug solution was dropped into the eyes each time for 7 days. The mice in the intravitreal injection group were anesthetized on the day of modeling and then administered by intravitreal injection, with a corresponding drug solution injected into each mouse using a micro-syringe (where 1 µL of the drug solution was injected into each eye in the aflibercept group, and 5 µL of the drug solution was injected into each eye in the HM-1 group). After the modeling, a fundus fluorescein angiography instrument system was used to conduct an FFA examination on day 3 after the modeling to evaluate a CNV leakage level. On day 7 after the modeling, the mice were sacrificed, administered FITC-dextran by intraventricular injection, and then subjected to RPE-choroid/sclera complex flat-mount fluorescence staining to detect changes in CNV area.

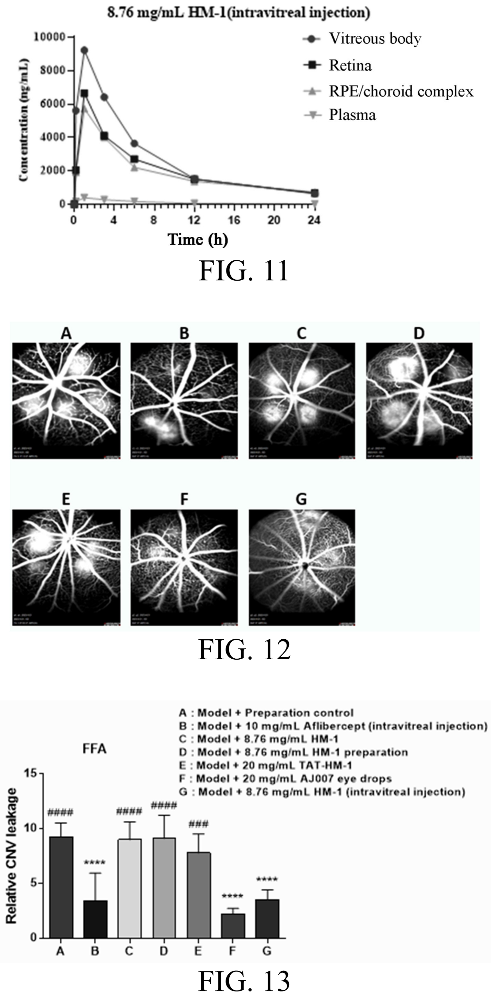

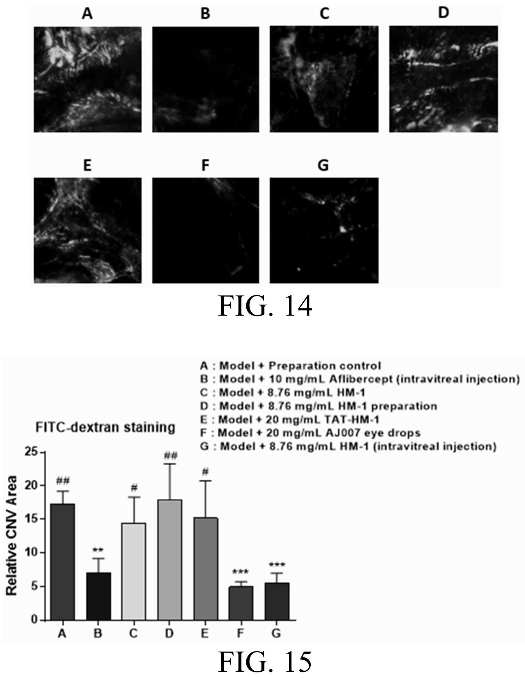

Experimental results and conclusions: FFA examination results show that the administration of HM-1, HM-1 preparation, and TAT-HM-1 by eye dropping cannot significantly improve laser-induced CNV leakage, while the intravitreal injection of HM-1 and the eye dropping of the AJ007 eye drops can significantly improve CNV leakage, which can achieve the efficacy on par with the intravitreal injection of positive drug aflibercept (FIG. 12 and FIG. 13), where the efficacy of the AJ007 eye drops after eye dropping is slightly better than that of the intravitreal injection of positive drug aflibercept. After the FITC-dextran injection, flat-mount staining results also show that the administration of HM-1, HM-1 preparation, and TAT-HM-1 by eye dropping cannot reduce the laser-induced CNV area, while the intravitreal injection of HM-1 and the eye dropping of the AJ007 eye drops can greatly reduce the CNV area, and the efficacy is even slightly better than that of the intravitreal injection of positive drug aflibercept (FIG. 14 and FIG. 15). According to comprehensive results of the FFA examination and the flat-mount staining after the FITC-dextran injection, it can be seen that neither HM-1, nor HM-1 prepared into a preparation, nor linking TAT to HM-1 have the effect of improving abnormal neovascularization of the wAMD mouse model. However, after linking TAT to HM-1 and then preparing a preparation to obtain the AJ007 eye drops, the efficacy in this classic w AMD model after the administration by eye dropping may be comparable to and even slightly better that of the intravitreal injection of HM-1 at the equimolar concentration, and the efficacy may also be on par with or even slightly better than that of the intravitreal injection of aflibercept, which is currently a treatment method with an optimal effect for wAMD clinically. Considering that the patient compliance of the administration route of eye dropping is significantly better than that of the administration route of intravitreal injection, the polypeptide eye drops of the present invention have good clinical potential, which provides a better option for the clinical treatment of wAMD patients and may generate considerable economic benefits.

According to the results of the above examples, by linking the cell-penetrating peptide TAT to the front end of the multifunctional fusion polypeptide VI (HM-1) sequence that has a higher ability to inhibit neovascularization and combining with an auxiliary material selected to prepare the eye drops in this application, the molecule is successfully optimized and upgraded. The ocular pharmacokinetics and laser-induced AMD mouse model efficacy results show that the AJ007 eye drops greatly increase tissue concentration and efficacy results in the retina and choroid compared with the original molecule of HM-1, and its efficacy is even slightly better than that of aflibercept, which is the best drug for wAMD clinically at present. Generally, the effect of 1+1>2 is achieved, and the effect of the eye drops is significantly better than that of eye drops linked to cell-penetrating peptides in other comparison documents. Additionally, considering that a clinical administration route of aflibercept is intravitreal injection, which is an invasive method, it is obvious that a non-invasive eye dropping method of the polypeptide eye drops is safer and more convenient for patients, which avoids various serious side effects caused by the intravitreal injection and has better clinical treatment potential.

## Claims

1. A fusion polypeptide AJ007, wherein an amino acid sequence of the fusion polypeptide is shown in SEQ ID NO: 2.

2. Use of the fusion polypeptide AJ007 according to claim 1 in preparation of a medicament for preventing or treating neovascular diseases in the posterior segment of ocular.

3. The use according to claim 2, wherein the neovascular diseases in the posterior segment of ocular comprise one or more of the following diseases: wet age-related macular degeneration, retinal vein occlusion, diabetic retinopathy, and retinopathy of prematurity.

4. The use according to claim 3, wherein the neovascular disease in the posterior segment of ocular comprises the wet age-related macular degeneration.

5. The use according to any one of claims 2-4, wherein the medicament for preventing or treating neovascular diseases in the posterior segment of ocular at least comprises the fusion polypeptide AJ007 and/or a pharmaceutically acceptable carrier thereof.

6. A polypeptide eye drop for preventing or treating neovascular diseases in the posterior segment of ocular, comprising the fusion polypeptide AJ007 according to claim 1 or a pharmaceutically acceptable salt form thereof, and being combined with an auxiliary material.

7. The polypeptide eye drop for preventing or treating neovascular diseases in the posterior segment of ocular according to claim 6, comprising the following components per 100 mL:
0-50% w/v of the fusion polypeptide AJ007 or a pharmaceutically acceptable salt form thereof;
5-10% w/v of 2-hydroxypropyl-β-cyclodextrin;
0.1-5% w/v of polysorbate 80;
0.05-5% w/v of tyloxapol;
0.05-10% w/v of poloxamer 407;
0-1% w/v of sodium dihydrogen phosphate;
0-0.005% w/v of a preservative;
0-1% w/v of an osmotic pressure regulator for regulating an osmotic pressure to 250-350 mOsm/kg; and
0-1% w/v of a pH regulator for regulating a pH to 6.0-8.0.

8. The polypeptide eye drop for preventing or treating neovascular diseases in the posterior segment of ocular according to claim 7, wherein the pharmaceutically acceptable salt form of the fusion polypeptide AJ007 comprises one or more of an acetate form, a trifluoroacetate form, a hydrochloride form, an ammonium salt form, a sodium salt form, a dihydroxynaphthalate form, a citrate form, and a salicylate form;
and/or the preservative comprises one or more of benzalkonium chloride, a benzalkonium bromide cationic surfactant, benzyl alcohol, chlorobutanol, and sorbic acid;
and/or the osmotic pressure regulator comprises one or more of sodium chloride, boric acid, and glucose;
and/or the pH regulator comprises one or more of a phosphate buffer solution, a boric acid buffer solution, hydrochloric acid, and sodium hydroxide.

9. The polypeptide eye drop for preventing or treating neovascular diseases in the posterior segment of ocular according to claim 8, wherein the pharmaceutically acceptable salt form of the fusion polypeptide AJ007 comprises the acetate form; the preservative comprises the benzalkonium chloride; the osmotic pressure regulator comprises the sodium chloride; and the pH regulator comprises the sodium hydroxide.

10. A method for preparing the polypeptide eye drop for preventing or treating neovascular diseases in the posterior segment of ocular according to any one of claims 6-9, comprising the following steps:
(1) preparation of an auxiliary material solution: weighing ultrapure water, sequentially adding specified amounts of the sodium dihydrogen phosphate, the benzalkonium chloride, the polysorbate 80, the poloxamer 407, and the tyloxapol, stirring continuously until complete dissolution, and filtering with a 0.22 µm filter membrane to obtain a solution serving as solution A for later use;
(2) preparation of a 2-hydroxypropyl-β-cyclodextrin solution: weighing the solution A, placing the same in a water bath stirring pot, heating up to 60°C, starting stirring, adding the 2-hydroxypropyl-β-cyclodextrin in several portions, and after complete dispersion and dissolution, taking out a solution and cooling to serve as solution B for later use;
(3) regulation of pH and osmotic pressure: detecting a pH and an osmotic pressure of the solution B, regulating the pH and the osmotic pressure using the pH regulator and the osmotic pressure regulator, and filtering with a 0.22 µm filter membrane to obtain a solution serving as solution C for later use;
(4) preparation of the polypeptide eye drops: weighing and adding the fusion polypeptide AJ007 or a pharmaceutically acceptable salt form thereof to the solution C, and shaking continuously to completely disperse and dissolve the fusion polypeptide AJ007 or a pharmaceutically acceptable salt form thereof to obtain the polypeptide eye drops.
